# NEUE EUROPÄISCHE PATENTSCHRIFT

(11) **EP 0 370 273 B2**
(45) Veröffentlichungstag und Bekanntmachung des Hinweises auf die Entscheidung über den Einspruch: **12.07.2000**
(45) Hinweis auf die Patenterteilung: 24.03.1993
(21) Anmeldenummer: 89120175.8
(22) Anmeldetag: 31.10.1989
(51) Int. Cl.: C07C 43/11, C07C 43/178, C07C 41/03, C11D 1/72

(54) **Fettalkoholethoxylate mit verbessertem Kälteverhalten**
Fatty alcohol ethoxylates having low temperature characteristics
Ethoxylates d'alcools gras à caractéristiques modifiées à basse température

(30) Priorität: 09.11.1988 DE 3837947
(43) Veröffentlichungstag der Anmeldung: 30.05.1990
(73) Patentinhaber: Cognis Deutschland GmbH, 40589 Düsseldorf (DE)
(72) Erfinder: Schmid, Karl-Heinz, Dr., D-4020 Mettmann (DE); Kubersky, Hans Peter, Dr., D-5650 Solingen 1 (DE); Demmering, Günter, Dr., D-5650 Solingen-Gräfrath (DE); Meffert, Alfred, Dr., D-4019 Monheim (DE)

(56) Entgegenhaltungen:
- DE-A- 2 513 377
- DE-C- 865 741
- GB-A- 1 029 502
- US-A- 2 094 127
- US-A- 3 193 586
- CHEMICAL ABSTRACTS, Band 102, Nr. 12, 25. März 1985, Seite 85, Zusammenfassung Nr. 97323t, Columbus, Ohio, US; & PL-A-116 074
- CHEMICAL ABSTRACTS, Band 90, Nr. 10, 5. März 1979, Seite 88, Zusammenfassung Nr. 73616y, Columbus, Ohio, US; & PL-A-93 875
- "Fettalkohole - Rohstoffe, Verfahren und Verwendung", Henkel KGaA, Düsseldorf, 1981, S. 18-19, 22, 25, 29, 130-142
- "Organische Chemie", L.F. Fieser et al, 1965, S. 1206-1208

## Beschreibung

Alkyl- bzw. Alkylaryl-polyglykolether als nichtionogene Tenside haben im letzten Jahrzehnt als Rezepturbestandteile von Wasch- und Reinigungsmitteln sowie als Bestandteile von Emulgatoren in kosmetischen oder technischen Präparaten eine immer stärkere Bedeutung gewonnen. Grundlage für die Synthese solcher nichtionogenen Tenside sind Alkylphenole, Oxoalkohole nach dem Hochdruck-(Roelen-Prozeß) bzw. Niederdruck-(Shop-Prozeß) Hydroformulierungsverfahren, Alkohole nach dem Ziegler-Verfahren sowie Fettalkohole, wie sie z. B. durch Hydrierung von natürlichen Ölen und Fetten bzw. aus den daraus hergestellten nativen Fettsäuren oder deren Methylestern gewonnen werden.

Alkylpolyglykolether auf Basis der Oxoalkohole sowie der Ziegler- und Fettalkohole erhalten aufgrund ihrer günstigeren ökotoxischen Eigenschaften im Vergleich zu den Alkylphenolpolyglykolethern immer stärkere Bedeutung. Da die meisten technischen Emulgatoren in Flüssigformulierungen eingesetzt werden, war eine der wichtigsten Anforderungen an nichtionogene Tenside schon immer ein möglichst niedriger Stockpunkt. Diese Nachfrage nach nichtionogenen Tensiden mit ausreichend gutem Kälteverhalten hat sich in den letzten Jahren insbesondere auch dadurch verstärkt, daß Flüssigformulierungen in Produktbereichen Eingang gefunden haben, wo ehemals nur Pulverformulierungen bekannt waren. Zu nennen ist hier vor allem der Bereich der Wasch- und Reinigungsmittel insbesondere der Textilwaschmittel.

Bezüglich des gewüschten guten Kälteverhaltens sind im Bereich der Alkylpolyglykolether die Ethoxylate von in ihrer Grundstruktur verzweigten Oxoalkoholen denen auf Basis linearer Alkohole - insbesondere Ziegler-Alkohole und Fettalkohole - bei gleicher Kohlenstoffkettenlänge des Alkylteils und gleicher Zahl an Ethylenoxideinheiten im Molekül überlegen. Bekanntlich liegt der Stockpunkt eines Alkohols wie auch des daraus hergestellten Ethoxylats um so tiefer, je verzweigter die Struktur des Alkohols ist.

Besondere anwendungstechnische Bedeutung kommt besonders Alkylethoxylaten mit im Mittel etwa 5 bis 10 Mol Ethylenoxid, bezogen auf 1 Mol Alkohol zu, wobei hier der Alkohol insbesondere eine Kohienstoffkettenlänge von etwa 12 bis 15 aufweist Verbindungen dieser Art zeichnen sich durch besonders gutes Wasch-, Netz-, Emulgier- und Reinigungsvermögen aus. Verbindungen dieser Art auf Basis heute verfügbarer Fettalkohole nativen Ursprungs vergleichbarer Kohlenstoffkettenlängen und gleichem Anteil von Ethylenoxid im Molekül sind bezüglich des Kälteverhaltens den entsprechenden Verbindungen auf Basis von Oxoalkoholen unterlegen. Die Erfindung strebt im Vergleich zur heute üblichen Bewältigung dieses Problems eine technisch bessere Lösung an.

Als natürliche Rohstoffquellen für einen Fettalkohol, der die zuvor beschriebene anwendungstechnisch notwendige Kohienstoffkettenlänge C 12 bis C 14 aufweist, kommen insbesondere Kokosöl, Palmkernöl und Babassuöl in Frage. Nachteilig an diesen Ölen ist aber, daß die daraus gewonnenen Fettalkohole - besonders bei Palmkernöl und Babassuöl - noch erhebliche Mengen an unerwünschten C 16/C 18-Anteilen enthalten, die bei den daraus hergestellten Ethoxylaten zu einem unbefriedigenden Kälteverhalten führen.

Will man nach der bisherigen technischen Praxis beispielsweise ein Alkylethoxylat mit im Mittel 7 Mol Ethylenoxid pro Mol Alkohol auf Basis eines Kokosalkohols herstellen, das über ein ähnliches Kälteverhalten verfügt wie das entsprechende Ethoxylat auf Basis eines Oxoalkohols der Kohlenstoffkettenlänge C 12 bis C 15, so war man bisher gezwungen, aus dem ex Kokosöl gewonnenen Alkohol den Anteil an C 16/18 durch fraktionierende Destillation zu entfernen. Dies ist nicht nur wegen der damit verbundenen Kosten für die Destillation teuer, der aus dem Kokosfettalkohol abgetrennte C 16/C 18-Alkohol ist mit den aus dem wesentlich billigeren Talg gewinnbaren Fettalkoholfraktionen identisch und dementsprechend im Vergleich zum Kokosalkohol stark abgewertet. Der entsprechende Materialverlust durch Abtrennen des C 16/C 18-Anteils beträgt bei einem Fettalkohoigemisch ex Kokosöl ca. 20 Gew.-% und bei einem Fettalkoholgemisch ex Palmkernöl ca. 25 Gew.-%.

Aufgabe der Erfindung war es, auf Basis von bevorzugt rein pflanzlichen Ölen bzw. Fetten, Ethoxylate von Fettalkoholgemischen, zur Verfügung zu stellen, die bezüglich ihres Kälteverhaltens mit den entsprechenden Materialien auf Basis von Oxoalkoholen vergleichbar sind und gleichwohl die aus anwendungstechnischen Gründen geforderten Kohlenstoffkettenlänge aufweisen.

Bei der Gewinnung von Fettalkoholen bzw. Fettalkoholfraktionen aus Fetten und/oder Ölen pflanzlichen Ursprungs werden nach der bisherigen Praxis die aus den pflanzlichen Ausgangsmaterlalien gewonnenen Fettsäuregemische als Fettsäuremethylester unter Verfahrenabedingungen hydrierend zu den entsprechenden Alkoholen reduziert, bei denen nicht nur die Reduktion der Carboxylfunktion, sondern auch die weitgehende Sättigung kettenständiger olefinischer Doppelbindungen stattfindet. Typische Beispiele für das bisherige Herstellungsverfahren solcher Fettalkoholfraktionen ist die Behandlung von Fettsäuremethyiestern ex Kokos-, Palmkern- und Babassuöl bei 200 bis 250 °C und Wasserstoffdrucken von 250 bis 300 bar über einem aus Kupfer- und Chromoxid bestehenden Katalysator, vergleiche hierzu beispielsweise H. Baumann, Angew. Chem. 100 (1988) 50 sowie U. R. Kreutzer, J. Amer. Oil Chem. Soc. 61 (1984), 343.

Die Lehre der Erfindung geht von der Feststellung aus, daß aus den gleichen pflanzlichen kohstoffen Fettalkohole und aus diesen Fettalkoholen hergestellte Ethoxylate mit wesentlich besserem Kälteverhalten hergestellt werden können, wenn man die kettenständige partiell olefinische Struktur dieser Ausgangsmaterialien so wenig wie möglich verändert und sich stattdessen auf eine möglichst selektive Reduktion der Carboxyfunktion beschränkt. Die in dieser Weise anfallenden Fettalkoholfraktionen. die in ihrer Zusammensetzung der Kohlenwasserstoffreste dem Typ der eingesetzten Pflanzenöle bzw. den dann vorliegenden Fettsäuregemischen möglichst weitgehend gleichen, zeichnen sich durch ein deutlich verbessertes Kälteverhalten aus. Wichtiger ist allerdings, daß in nicht vorhersehbarer Weise die zuvor angesprochenen Ethoxylate des aus beispielsweise wasch- und reinigungstechnischen Gründen erforderlichen mittleren Ethoxylierungsgrades bezüglich des Kälteverhaltens Produkteigenschaften besitzen, die denen der bisher überlegenen Oxoalkoholderivate wenigstens entsprechen.

Es ist an sich bekannt, durch Auswahl geeigneter Verfahrensbedingungen und insbesondere geeigneter Katalysatoren die hydrierende Reduktion ungesättigter Fettsäuren unter weitgehend selektiver Umwandlung der Carboxylfunktion und unter Erhaltung der kettenständigen Doppelbindungen der Ausgangsverbindungen zu ungesättigten Alkoholen vorzunehmen. So kann beispielsweise ausgehend von Ölsäure bzw. Ölsäuremethylester bei Verwendung spezieller zinkhaltiger Katalysatoren Oleytalkohol gewonnen werden, vergleiche hierzu beispielsweise die bereits zitierte Literatur H. Baumann a.a.O. und U. R. Kreutzer a.a.O. Zusätzlich sei verwiesen auf die deutsche Patentschrift 25 13 377 sowie die darin zitierten älteren deutschen Patentschriften 865 741 und 965 236. Der Kern der erfindungsgemäßen Lehre liegt demgegenüber in der bisher nicht bekannten Erkenntnis, daß - ausgehend von den Pflanzenölen bzw. -fetten bevorzugt rein natürlichen Ursprungs-Reaktionsprodukte der geschilderten Art erhalten werden können, die in ihrem Leistungsniveau vergleichbaren Produkten auf Basis von Oxoalkoholen mindestens entsprechen. Der auf natürliche nachwachsende Rohstoffe basierten Chemie eröffnet sich damit im hier diskutierten Teilaspekt eine wichtige Erweiterung im Sinne der von der Praxis heute gestellten Anforderungsprofile.

Aus "Fettalkohole, Rohstoffe, Verfahren und Verwendung", Hrsg. von Henkel KGaA, Düsseldorf, 1981, S. 18, 19, 22, 25, 29, 130 bis 142, sind zwar prinzipiell Fettalkoholethoxylate natürlichen Ursprungs und Flüssigformulierungen mit diesen nichtionischen Tensiden bekannt, aber diese weisen aufgrund des gesättigten C_{16/18}-Anteils ein unbefriedigendes Kälteverhalten auf Zwar sind in dieser Druckschrift physikalische Angaben zur Beschaffenheit bei Raumtemperatur im Sinne von flüssiger oder fester Konsistenz gegeben, aber diese lassen keine Rückschlüsse auf das Kälteverhalten im Sinne eines Auftauverhaltens nach Verfestigung durch Unterkühlung zu.

Gegenstand der Erfindung sind dementsprechend Ethoxylate von Fettalkoholgemischen R¹-OH natürlichen, insbesondere rein pflanzlichen Ursprungs mit verbessertem Kälteverhalten und der allgemeinen Formel R¹O(CH₂CH₂O)ₓH, in der x eine Zahl von 2 bis 10, bevorzugt von 3 bis 9, bedeutet und R¹ für Gemische von gesättigten und olefinisch ungesättigten Fettalkohl-Kohlenwasserstoffresten der Bereiche C 6 bis 20 oder C 12 bis 20 steht, die für den Kettenlängenbereich R¹ =C 6 bis 20 der nachfolgenden Spezifikation 1 und für den Kettenlängenbereich R¹ =C 12 bis 20 der nachfolgenden Spezifikation II entsprechen:

### Spezifikation I

| | strukturanalog zu | Gew.-% |
|---|---|---|
| C 6 | Capronsäure | 0,1 - 0,6 |
| C 8 | Caprylsäure | 2,5 - 10 |
| C 10 | Caprinsäure | 2,5 - 14 |
| C 12 | Laurinsäure | 37 - 52 |
| C 14 | Myristinsäure | 10 - 20 |
| C 16 | Palmitinsäure | 6 - 10 |
| C 18 | Stearinsäure | 1 - 5 |
| C 18' | Ölsäure | 5 - 23 |
| C 18'' | Linolsäure | 1 - 4 |
| C 18''' | Linolensäure | 0,1 - 1 |
| C 20 | Arachinsäure | 0,1 - 1 |

### Spezifikation II

| | strukturanalog zu | Gew.-% |
|---|---|---|
| C 12 | Laurinsäure | 39 - 69 |
| C 14 | Myristinsäure | 10 - 27 |
| C 16 | Palmitinsäure | 6 - 14 |
| C 18 | Stearinsäure | 1 - 7 |
| C 18' | Ölsäure | 6 - 31 |
| C 18'' | Linolsäure | 1 - 6 |
| C 18''' | Linolensäure | 0,1 - 2 |
| C 20 | Arachinsäure | 0,1 - 2 |

Besondere Bedeutung kann den Ethoxylaten von Fettalkolgemischen der Spezifikation I zukommen, wenn das Verhältnis des einfach olefinisch ungesättigten Kohlenwasserstoffrestes mit 18 C-Atomen zur Summe der einfach und zweifach olefinisch ungesättigten Kohlenwasserstoffreste mit jeweils 18 C-Atomen größer ist als 78 %.

Bevorzugte Ethoxylate basieren auf Fettalkoholgemiche der zuvor angegebenen Spezifikationen, die durch selektive Hydrierung entsprechender Gemische von Fettsäuren natürlichen Ursprungs bevorzugt rein pflanzlichen Ursprungs bzw. durch die selektive Hydrierung von entsprechenden Estergemischen solcher Fettsäuren zugänglich sind. Besonders geeignete Ester für diese selektive Hydrierung sind entsprechende Ester mit monofunktionellen Alkoholen insbesondere niederen einwertigen Alkaholen mit 1 bis 5 C-Atomen. Besondere Bedeutung kommt hier bekanntlich dem Methylester und in gewissem Umfang auch noch dem Butylester zu. Die selektive Hydrierung wird unter möglichst weitgehender Beibehaltung der natürlichen Verteilung der gesättigten sowie der ein- und mehrfach olefinisch ungesättigten Kohlenwasserstoffreste im Einsatzmaterial durchgeführt Besonders geeignete Einsatzmaterlalien rein pflanzlichen Ursprungs sind Kokosöl. Palmkernöl, Babassuöl und Roccafett bzw. die daraus gewonnenen Fettsäure- und insbesondere die entsprechenden Fettsäuremethylestergemische.

Fettalkoholgemische der zuvor genannten Spezifikation I enthalten noch die niederen Fraktionen von C 6 bis C 10, die in bestimmten Einsatzgebieten geruchlich unerwünscht sein können. Die Erfindung umfaßt dementsprechend auch Ethoxylate von Alkoholgemishen der zuvor genannten Spezifikation II, die sich aus den Stoffgemischen der zuvor genannten Spezifikation I durch Abtrennung der C 6 bis C 10-Fraktion mühelos gewinnen lassen. Beide Fettalkoholgemische und insbesondere die daraus gewonnenen erfindungsgemäß beschriebenen Ethoxylate zeichnen sich jedoch durch ihren beträchtlichen Gehalt insbesondere an C 16/C 18-Komponenten aus. Der erfindungsgemäß vorgeschriebene Gehalt dieser Fraktion an ein- und mehrfach olefinisch ungesättigten Komponenten stellt gleichwohl das angestrebte Ziel der mit den Oxoalkoholen bzw. deren Derivaten vergleichbaren Kältestabilität sicher, ohne daß substantielle Materialverluste des nachwachsenden Einsatzmaterials in Kauf zu nehmen wären. Auch in den sonstigen anwendungstechnischen Eigenschaften sind die hier interessierenden Ethoxylate voll befriedigend.

Die Herstellung der Ethoxylate aus den erfindungsgemäß definierten Fettalkoholgemischen erfolgt in an sich bekannter Weise durch Umsetzung der Alkoholgemische mit Ethylenoxid entweder unter alkalischer oder saurer Katalyse. Zu dieser an sich bekannten Methodik der Herstellung insbesondere entsprechender nichtionischer Tenside existiert eine umfangreiche Literatur. Im technischen Verfahren ist weit verbreitet die alkalische Katalyse, wobei im allgemeinen Natriummethylat aber auch Nataiumhydroxid, Natrium, Natriumethylat, Kaliumhydroxid usw. als Katalysatoren verwendet werden. Durch die Ringöffnung verläuft die Reaktion exotherm. Die Reaktionstemperatur liegt beispielsweise im Bereich von etwa 135 bis 150 °C. Das Reaktionsprodukt wird anschließend mit anorganischen oder organischen Säuren. z. B. mit Essigsäureanhydrid, Phosphorsäure oder Kohlendioxid neutralisiert. Das entstehende Ethoxylierungsprodukt ist nicht einheitlich, man erhält vielmehr Polymerhomologe. Je nach Art der Katalyse ist dabei die Verteilungskurve unterschiedlich. Neben der hier beschriebenen üblichen diskontinuierlichen Ethoxylierung gibt es auch kontinuierliche Verfahren. Die Reaktionsbedingungen können dabei wesentlich drastischer gewählt werden. So kann man beispielsweise bei 50 bis 70 atm und 250 bis 350 °C arbeiten. Zur einschlägigen Literatur wird beispielsweise verwiesen auf die Veröffentlichung Dr. W. Wirth "Tenside, Detergents" 12 (1975) 245 bis 254 sowie die dort zitierte Literatur.

Die Erfindung betrifft schließlich in einer weiteren Ausführungsform die Verwendung von Ethoxylaten ausgewählter Fettalkoholgemische natürlichen, bevorzugt rein pflanzlichen Ursprungs mit der angegebenen allgemeinen Formel R¹-O-(CH₂CH₂O)ₓ-H, in der R¹ den zuvor angegebenen Spezifikationen I oder II entspricht und x im Mittel eine Zahl von 2 bis 10, bevorzugt von 3 bis 9 bedeutet, als nichtionische Tensidkomponente in Flüssigformulierungen. Ethoxylate der hier geschilderten Art zeichnen sich insbesondere im Bereich der Raumtemperatur durch ihr Vorliegen als homogene flüssige Phase auch mit guten Fließeigenschaften aus. Auch wenn Tensidgemische der hier betroffenen Art durch Unterkühlung verfestigt werden, so schmelzen solche Materialien bei Einstellung von Raumtemperatur in kurzer Frist wieder zur leicht fließfähigen homogenen Flüssigphase auf. Für die Anwendung dieser Materialien als nichtionische Tensidkomponente ergeben sich damit in der täglichen Praxis auf vielen Einsalzgebieten beträchtliche Vorteile. Es ist nicht nur die bessern Handhabbarkeit sichergestellt. Die bisher üblichen nichtionische Tenside der hier betroffenen Art auf Basis von Pflanzenfetten waren insbesondere bei höheren EO-Gehalten innerhalb des geschilderten Bereichs dadurch gefährdet, daß eine partielle Verfestigung des Reaktionsproduktes bei längerer Lagerung unter Raumtemperatur zu beobachten ist. Solche Reaktionsprodukte können durchaus noch fließfähig sein, ihre absatzweise Verarbeitung aus einem Vorratsbehälter macht aber begreiflicherweise Schwierigkeiten. Es bedarf vorder Entnahme eines Materialanteils jeweils einer sorgfältigen Homogenisierung des Behälterinhaltes, um Verschiebungen in der Zusammensetzung des komplexen Reaktionsproduktes sicher auszuschließen. Die erfindungsgemäß bevorzugten Ethoxylate liegen im Bereich der Raumtemperatur als homogene Flüssigphase vor, in der Entmischungsvorgänge von vorneherein ausscheiden. Eine mengenmäßig nicht eingeschränkte Bevorratung und anteilsweise Materialentnahme mit immer gleichbleibenden Produkteigenschaften sind dadurch sichergestellt

In einer besonderen Ausführungsform verbindet die Lehre der Erfindung die neuen Maßnahmen zur Verbesserung der Kältebeständigkeit von Fettalkoholethoxylaten der geschilderten Art mit der Lehre der älteren Anmeldung P 3802027.0 (D 8083) der Anmelderin. Beschrieben wird in dieser älteren Anmeldung ein Verfahren zur Herstellung von Alkytpolyglykoiethern mit verbessertem Kälteverhalten durch Umsetzung von gesättigten und/oder ungesättigten, insbesondere geradkettigen Alkoholen mit Ethylenoxid bei erhöhten Temperaturen in Gegenwart von basischen Alkalimetallverbindungen als Katalysatoren und nachfolgende Neutralisation des alkalischen Katalysators mit organischen und/oder anorganischen Säuren. Das Kennzeichen der Lehre aus diesem älteren Schutzrecht liegt darin, daß man mit derart erhöhten Katalysatorkonzentrationen arbeitet daß in der Neutralisationastufe eine Salzfällung als ungelöste Feststoffphase auftritt und daß man dabei den Neutralisationsschritt dergestalt durchführt, daß man die Ausfällung der Salzphase in Gegenwart von im Reaktionsprodukt dispergierten feinteiligen Feststoffen vornimmt.

Diese Lehre der älteren Anmeldung geht dabei von der Aufgabe aus, Fettalkoholethylenoxidaddukte mit verbesserter Kältestabilität ohne Verschlechterung der anderen anwendungstechnischen Eigenschaften in einem technisch einfach durchführbaren Verfahren zugänglich zu machen. Die technische Lösung dieser Aufgabe geht von der Erkenntnis aus, daß man bei einem begrenzten Anheben der Menge der basischen Alkalimetallkatalysatoren im Ethoxylierungsschritt substantielle Verbesserungen der die Kältestabilität charakterisierenden Stoffparameter einstellen kann, ohne in unerwünschter Weise die anwendungstechnischen Eigenschaften wie Wasch- bzw. Netzvermögen, Emulgiervermögen und dergleichen zu beeinträchtigen. Dabei muß allerdings die Menge des basischen Katalysators über den Grenzbetrag hinaus erhöht werden, der die Löslichkeit des durch Neutralisation anfallenden Satzes im Ethoxylierungsprodukt kennzeichnet. Zur Lösung dieser Schwierigkeit wird dementsprechend in der älteren Anmeldung vorgeschlagen, die Katalysatorneutralisation in Gegenwart von feinteiligen Feststoffen vorzunehmen, die im Reaktionsgemisch möglichst homogen verteilt sind. Überraschenderweise hat sich gezeigt daß durch diese Maßnahme Verkrustungen an Apparateund/oder Leitungsinnenteilen vollständig verhindert werden können und daß weiter hin die Filtration des Reaktionsproduktes in konventionellen Verfahren ohne Schwierigkeiten möglich ist

Das Ergebnis des Arbeitens mit er höhten Katalysatormengen im Polyethoxytierungsschritt ist die Herstellung von Alkylpolyglykolether-Reaktionsprodukten mit eingeengter Homologenverteilung. Solche eingeengten Homologenverteilungen führen - insbesondere durch Einschränkung des Anteils an höheren Homologen - im allgemeinen zur Absenkung der Kälte-charakteristischen Produktwerte. In der hier geschilderten besonderen Ausführungsform der vorliegenden Erfindung wird von dieser Maßnahme der älteren Anmeldung gemäß P 3802027.0 (D 8083) zusätziich Gebrauch gemacht Das Ergebnis des erfindungsgemäßen Handelns sind damit dann Fettalkoholethoxylate mit nochmals verbesserten und damit insgesamt optimierten Kälteeigenschaften.

Zu den Einzelheiten dieses Arbeitens mit einem bevorzugt beschränkt erhöhten Betrag an alkalischen Katalysatoren im Schritt Fettalkoholethoxylierung wird auf die Angaben der genannten älteren Anmeldung P 3802027.0 (D 8083) verwiesen. Zur Vervollständigung der Erfindungsofftenbarung auch in Rahmen der vorliegenden Beschreibung werden im nachfolgenden die essentielien Elemente dieses älteren Vorschlages zusammengefaßt.

Als feinteilige Feststoffe zur Ausfällung der Salzphase in Reaktionsprodukt werden bevorzugt organische und/oder anorganische Filterhilfsmittel eingesetzt, die im Alkylpolyglykolether-Reaktionsprodukt dispergiert werden, bevor eine Auskristallisation der Salzphase auftritt. Die feinteiligen Filterhilfsmittel werden bevorzugt in Mengen von etwa 0,3 bis 2 Gew.-%, insbesondere in Mengen von etwa 0,5 bis 1 Gew.-% - bezogen jeweils auf den gebildeten Alkylpolyglykolether - eingesetzt. Als Filterhilfsmittel können insbesondere auch Gemische von anorganischem und organischem Material, vorzugsweise im Mengenbereich von etwa 3 : 1 bis 1 : 3 eingesetzt werden. Ein geeignetes anorganisches Flterhilfsmittel ist beispielsweise Kieselgur, organische Filterhilfsmittel sind beispielsweise Holzmehl und/oder feinpulvrige Cellulose.

Nach Abschluß der Ethoxylierung wird in Gegenwart der Filterhilfsmittel zunächst eine partielle Neutralisation, beispielsweise bis etwa pH 8 vorgenommen und dann eine Bleiche durch Zusatz von insbesondere Wasserstoffperoxid durchgeführt, woraufhin erst danach die volle Neutralisation des Reaktionsproduktes auf pH-Werte im Bereich von etwa 6,5 bis 7,5 vorgenommen wird. Bevorzugt wird weiterhin die Neutralisationsstufe und Ausfällung der Salzphase auf der Oberfläche des dispergierten Filterhilfamittels im Temperaturbereich von etwa 50 bis 110 °C vorgenommen.

Die zur Ethoxylierung der sterisch nicht gehinderten Alkohole eingesetzten Mengen an basischem Katalysator liegen bei diesem Verfahren in der Regel bei wenigstens etwa 0,5 Gew.-% - berechnet als Natriummethylat und bezogen auf das Gesamtgemisch aus Alkohol und Ethylenoxid - wobei Mengen im Bereich von etwa 0,5 bis 1,5 Gew.-% und insbesondere solche im Bereich von etwa 0,8 bis 1,1 Gew.-% besonders bevorzugt sein können. Die technische Lehre ist nicht auf das Arbeiten mit Natriummethylat eingeschränkt. Auch andere bekannte basische Alkalimetallkatalysatoren können in äquivalenten Mengenverhältnissen zur Verwendung kommen. Die Neutralisation des Katalysators im Reaktionsprodukt wird mit organischen und/oder anorganischen Säuren durchgeführt. Als Beispiele seien genannt Gluconsäure, Glycoisäure, Essigsäure, Ameisensäure, Benzoesäure, Milchsäure, Oxalsäure, Zitronensäure, Propionsäure, Phosphorsäure, Methansulfonsäure und/oder Diglykolsäure. Stark korrosive Säuren beispielsweise Schwefelsäure oder Salzsäure haben in der Praxis keine Bedeutung.

Nach Abschluß der Neutralisation wird das Ethoxylat, In dem Filterhilfsmittel und gebildetes Salz homogen verteilt sind, zur Filtrationsstufe gepumpt. Die Filtration kann sowohl über Durchflußfilter als auch über Filterpressen und über Drehfilter erfolgen. Besonders salzarme Ethoxylate erhält man, wenn die unter Stickstoff erfolgende Filtration bei höheren Temperaturen als 80 °C durchgeführt wird.

### Beispiele

Für die Herstellung der in den nachfolgenden Beispielen und Vergleichsbeispielen beschriebenen Fettalkoholethoxylaten werden die jeweils in den Beispielen angegebenen Mengen an Fettalkohol, Ethylenoxid und Katalysator in einem Autoklaven bei 170 bis 180 °C mit einem Reaktionsdruck von 2 bis maximal 5 bar ca. 3 bis 4 Stunden umgesetzt.

Nach Abschluß der Alkoxylierungsreaktion wird das Fettalkoholethoxylat in einen mit Rührer ausgestatteten Neutralisationsbehälter überführt und die Neutralisation des Produktes bei 90 °C mit Milchsäure vorgenommen, so daß das Reaktionsprodukt schließlich einen pH-Wert (1 % des Produktes in 99 % deionisiertem Wasser) von 6,5 bis 7,5 aufweist. In den Beispielen, in denen die für die Alkoxylierungsreaktion eingesetzte Menge Natriummethylat (bezogen auf die Gesamtmenge von Alkohol und Ethylenoxid) 0,5 Gew.-% überschreitet, wird das Alkoxylierungsprodukt nach Zugabe von maximal 1 Gew.-% Fiterhilfsmittel und anschließender Neutralisation mit Milchsäure filtriert

Die Bestimmung der Hydrophilie (Trübungspunkt, Trübungstemperatur, Trübungstitrationszahl) des Ethoxylats erfolgt nach DIN 53 917.

### Vergleichsbeispiel 1

Ein aus Palmkernöl durch Umesterung mit Methanol und Fraktionierung des Reaktionsprodukts gewonnener Fettsäuremethylester im Kettenlängenbereich C 12 bis C 18 wurde in einer kontinuierlich arbeitenden Hydrieranlage an einem Kupfer enthaltenden Festbettkatalysator bei einem Druck von 250 bar und einer Temperatur von 240 °C mit Wasserstoff zu einem Gemisch aus Fettalkohol des genannten C-Kettenlängenbereichs und Methanol umgesetzt. Nach Abdestillieren des Methanols wies der Fettalkohol die folgenden Kennzahlen

auf:
- Hydroxylzahl:: 269
- Verseifungazahl:: 1,1
- Säurezahl:: 0,02
- Jodzahl:: 0,12
- Erstarrungapunkt:: 23 °C

### Herstellung der Fettalkoholgemische

In einem zweiten Versuch wurde der gleiche Palmkernfettsäuremethylester in einer kontinuierlich arbeitenden Hydrieranlage an einem Chrom/Zink enthaltenden Festbettkatalysator hydriert Der Druck betrug 250 bar und die Temperatur 300 °C. Unter diesen Bedingungen blieben die in der Kohlenstoffkette des Methylesters vorliegenden Doppelbindungen erhalten, und es entstand ein Fettalkohol, der nach Abdestillieren des mitgebildeten Methanols die folgenden Kennzahlen aufwies:
- Hydroxylzahl:: 270,5
- Verseifungszahl:: 1,7
- Säurezahl:: 0,02
- Jodzahl:: 11,7
- Erstarrungspunkt:: 17 °C

Für die nachfolgenden Beispiele werden die vier Kokosalkohole "Typ I bis Typ IV" eingesetzt. Dabei entsprechen die Typen I und III der erfindungsgemäßen Definition, während die Typen II und IV weitgehend frei sind an olefinisch ungesättigten C 18-Alkoholen.

### Kokosalkohol (Typ I)

- Hydroxylzahl:: 267
- Jodzahl:: 12

| C-Kettenverteilung: | |
|---|---|
| C 12 | 53,5 |
| C 14 | 21,8 |
| C 16 | 10,9 |
| C 18 | 4,1 |
| C 18' | 9,7 |

### Kokosalkohol (Typ II)

- Hydroxylzahl:: 269
- Jodzahl:: 0,2

| C-Kettenverteilung: | |
|---|---|
| C 12 | 54,0 |
| C 14 | 22,5 |
| C 16 | 10,1 |
| C 18 | 13,3 |
| C 18' | 0,5 |

### Kokosalkohol (Typ III)

- Hydroxylzahl:: 266
- Jodzahl:: 15

| C-Kettenverteilung: | |
|---|---|
| C 12 | 52,0 |
| C 14 | 21,3 |
| C 16 | 9,0 |
| C 18 | 2,1 |
| C 18' | 15,6 |

### Kokosalkohol (Typ IV)

- Hydroxylzahl:: 265
- Jodzahl:: 0,3

| C-Kettenverteilung: | |
|---|---|
| C 12 | 54,0 |
| C 14 | 17,2 |
| C 16 | 8,5 |
| C 18 | 20,2 |
| C 18' | 0,1 |

### Beispiel 1

569,9 g Kokosalkohol (Typ I) wurden mit 830,1 g Ethylenoxid und 12,6 g Natriummethylat (30 %ig, in Methanol, entspricht 0,27 Gew.-% Natriummethylat bezogen auf die Gesamtmenge von Alkohol und Ethylenoxid) umgesetzt. Man erhielt ein Ethoxylat mit folgenden Kennzahlen:

| | |
|---|---|
| OHZ | 108 |
| % H₂O | 0,19 |
| Dichte (70 %) | 0,9462 |
| Trübungstemperatur (5 g in 25 g 25-%igem Butyldiglykol) | 79 °C |
| % Polyethylenglykol | 2,2 mit einem Molekulargewicht von 400 |

Das Produkt, das einem Fettalkohol-7EO-Addukt entspricht wurde auf - 10°C abgekühlt, bis es fest war. Danach wurde das Produkt 24 Stunden lang bei 22°C gelagert. Nach dieser Zeit war das Produkt flüssig.

### Vergleichsbeispiel 1

565,5 g Kokosalkohol (Typ II) wurden mit 834,5 g Ethylenoxid und 12,6 g Natriummethylat (30 %ig, in Methanol, entspricht 0,27 Gew.-% Natriummethylat bezogen auf die Gesamtmenge von Alkahol und Ethylenoxid) umgesetzt. Man erhielt ein Ethoxylat mit folgenden Kennzahlen:

| | |
|---|---|
| OHZ | 111 |
| % H₂O | 0,16 |
| Dichte (70 °C) | 0,9468 |
| Trübungstemperatur (5 g in 25 g 25-%igem Butyldiglykol) | 81 °C |
| % Polyethylenglykol | 2,1 mit einem Molekulargewicht von 800 |

Das Produkt, das einem Fettalkohol-7EO-Addukt entspricht, wurde auf - 10°C abgekühlt, bis es fest war. Danach wurde das Produkt 1 Woche lang bei 22 °C gelagert. Nach dieser Zeit war das Produkt immer noch fest.

### Beispiel 2

483,8 g Kokosalkohol (Typ I) wurden mit 916,2 g Ethylenoxid und 7,0 g Natriummethylat (30 %ig, in Methanol, entspricht 0,15 Gew.-% Natriummethylat bezogen auf die Gesamtmenge von Alkohol und Ethylenoxid) umgesetzt. Man erhielt ein Ethoxylat mit folgenden Kennzahlen:

| | |
|---|---|
| OHZ | 95 |
| % H₂O | 0,14 |
| Dichte (70 °C) | 0,9647 |
| Trübungstemperatur (1 %ig in H₂O) | 78 °C |
| % Polyethylenglkyol | 2,5 mit einem Molekulargewicht von 1000 |

Das Produkt, das einem Fettalkohol-9EO-Addukt entspricht, wurde auf - 10°C abgekühlt, bis es fest war. Danach wurde das Produkt 24 Stunden lang bei 25°C gelagert. Nach dieser Zeit war das Produkt bereits flüssig.

### Vergleichsbeispiel 2

483,8 g Kokosalkohol (Typ II) wurden mit 916,2 g Ethylenoxid und 7,0 g Natriummethylat (30 %ig, in Methanol, entspricht 0,15 Gew.-% Natriummethylat bezogen auf die Gesamtmenge von Alkohol und Ethylenoxid) umgesetzt. Man erhielt ein Ethoxylat mit folgenden Kennzahlen:

| | |
|---|---|
| OHZ | 92 |
| % H₂O | 0,20 |
| Dichte (70 °C) | 0,9649 |
| Trübungstemperatur (1 %ig in H₂O) | 79 °C |
| % Polyethylenglykol | 2,3 mit einem Molekulargewicht von 1100 |

Das Produkt, das einem Fettalkohol-9EO-Addukt entspricht, wurde auf - 10°C abgekühlt, bis es fest war. Danach wurde das Produkt 1 Woche lang bei 25°C gelagert. Nach dieser Zeit war das Produkt immer noch fest.

### Beispiel 3

567,5 g Kokosalkohol (Typ III) wurden mit 832,5 g Ethylenoxid und 12,6 g Natriummethylat (30 %ig, in Methanol, entspricht 0,27 Gew.-% Natriummethylat bezogen auf die Gesamtmenge von Alkohol und Ethylenoxid) umgesetzt. Man erhielt ein Ethoxylat mit folgenden Kennzahlen:

| | |
|---|---|
| OHZ | 110 |
| % H₂O | 0,21 |
| Dichte (70 °C) | 0,9463 |
| Trübungstemperatur (5 g in 25 g 25-%igem Butyldiglykol) | 82 °C |
| % Polyethylenglykol | 2,0 mit einem Molekulargewicht von 500 |

Das Produkt, das einem Fettalkohol-7EO-Addukt entspricht, wurde auf - 10°C abgekühlt, bis es fest war. Danach wurde das Produkt 24 Stunden lang bei 22°C gelagert. Nach dieser Zeit war das Produkt flüssig.

### Vergleichsbeispiel 3

567,5 g Kokosalkohol (Typ IV) wurden mit 832.5 g Ethylenoxid und 12,6 g Natriummethylat (30 %ig, in Methanol, entspricht 0,27 Gew.-% Natriummethylat bezogen auf die Gesamtmenge von Alkohol und Ethytenoxid) umgesetzt. Man erhielt ein Ethoxylat mit folgenden Kennzahlen:

| | |
|---|---|
| OHZ | 109 |
| % H₂O | 0,25 |
| Dichte (70 °C) | 0,9464 |
| Trübungstemperatur (5 g in 25 g 25-%igem Butyldiglykol) | 81 °C |
| % Polyethylenglykol | 2,1 mit einem Molekulargewicht von 700 |

Das Produkt, das einem Fettalkohol-7EO-Addukt entspricht, wurde auf - 10°C abgekühlt, bis es fest war. Danach wurde das Produkt 24 Stunden lang bei 22 °C gelagert. Nach dieser Zeit war das Produkt immer noch fest.

### Vergleichsbeispiel 4

Ein Oxoalkohol der Kohlenstoffkettenlänge C 14/C 15 mit 7 Mol Ethylenoxid pro Mol Alkohol wurde auf - 10 °C abgekühlt, bis er fest war. Danach wurde das Produkt 4 Wochen lang bei 22 °C gelagert. Das Produkt war nach dieser Zeit immer noch fest.

### Vergleichsbeispiel 5

Ein Oxoalkohol der Kohlenstoffkettenlänge C 12/C 13 mit 7 Mol Ethylenoxid pro Mol Alkohol wurde auf-10 °C abgekühlt, bis er fest war. Danach wurde das Produkt 24 Stunden lang bei 22°C gelagert. Das Produkt war nach dieser Zeit flüssig.

## Patentansprüche

1. Fettalkoholethoxylate natürlichen Ursprungs der allgemeinen Formel (**I**) 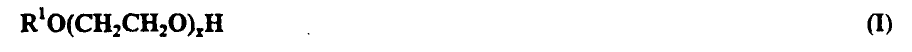 in der x für eine Zahl im Bereich von 2 bis 10 steht und R¹ für Gemische von gesättigten und olefinisch ungesättigten Fettalkohol-Kohlenwasserstofftesten der Bereiche C₆₋₂₀ oder C₁₂₋₂₀ steht, die für den Kettenlängenbereich R¹ = C₆₋₂₀ der nachfolgenden Spezifikation I und für den Kettenlängenbereich R¹ = C₁₂₋₂₀ der nachfolgenden Spezifikation II entsprechen:
| | **strukturanalog zu** | **I Gew.-%** | **II Gew.-%** |
|---|---|---|---|
| C6 | Capronsäure | 0,1-0,6 | |
| C8 | Caprylsäure | 2,5-10 | |
| C10 | Caprinsäure | 2,5-14 | |
| C12 | Laurinsäure | 37-52 | 39-69 |
| C14 | Myristinsäure | 10-20 | 10-27 |
| C16 | Palmitinsäure | 6-10 | 6-14 |
| C18 | Stearinsäure | 1-5 | 1-7 |
| C18' | Olsäure | 5-23 | 6-31 |
| C18'' | Linolsäure | 1-4 | 1-6 |
| C18''' | Linolensäure | 0,1-1 | 0,1-2 |
| C20 | Arachinsäure | 0,1-1 | 0,1-2 |

2. Fettalkoholethoxylate nach Anspruch 1, **dadurch gekennzeichnet,** daß sie durch selektive Hydrierung von Pflanzenfetten und -ölen bzw. entsprechender Gemische von Fettsäuren bevorzugt rein pflanzlichen Ursprungs und/oder deren Ester mit niederen Alkoholen unter möglichst weitgehender Beibehaltung der natürlichen Verteilung der gesättigten sowie der ein- und mehrfach olefinisch ungesättigten Kohlenwasserstoffreste und anschließende Ethoxylierung erhalten werden.

3. Fettalkoholethoxylate nach den Ansprüchen 1 und 2, **dadurch gekennzeichnet,** daß sie durch Umsetzung der entsprechenden Fettalkohole bei erhöhter Temperatur und in Gegenwart derart erhöhter Mengen an basischen Alkalimetallverbindungen als Katalysatoren hergestellt worden sind, daß in der Neutralisationsstufe eine Salzfällung als ungelöste Feststoffphase auftritt, wobei der Neutralisationsschritt dergestalt durchgeführt worden ist, daß die Ausfällung der Salzphase in Gegenwart von im Reaktionsprodukt dispergierten feinteiligen Feststoffen vorgenommen wird.

4. Verwendung von Fettalkoholethoxylaten nach Anspruch 1 als nichtionische Tensidkomponente in Flüssigformulierungen.

## Claims

1. Fatty alcohol ethoxylates of natural origin corresponding to general formula (I):
R¹O(CH₂CH₂O)ₓH (I)
in which x is a number of 2 to 10 and R¹ stands for mixtures of saturated and olefinically unsaturated C₆₋₂₀ or C₁₂₋₂₀ fatty alcohol hydrocarbon residues which, for the chain length range R¹ = C₆₋₂₀, correspond to specification I below and, for the chain length range R¹ = C₁₂₋₂₀, correspond to specification II:
| | Structurally analogous to | I % by weight | II % by weight |
|---|---|---|---|
| C6 | Caproic acid | 0.1-0.6 | |
| C8 | Caprylic acid | 2.5-10 | |
| C10 | Capric acid | 2.5-14 | |
| C12 | Lauric acid | 37-52 | 39-69 |
| C14 | Mristic acid | 10-20 | 10-27 |
| C16 | Palmitic acid | 6-10 | 6-14 |
| C18 | Stearic acid | 1-5 | 1-7 |
| C18' | Oleic acid | 5-23 | 6-31 |
| C18'' | Linoleic acid | 1-4 | 1-6 |
| C18''' | Linolenic acid | 0.1-1 | 0.1-2 |
| C20 | Arachic acid | 0.1-1 | 0.1-2 |

2. Fatty alcohol ethoxylates as claimed in claim 1, characterized in that they are obtained by selective hydrogenation of vegetable fats and oils or corresponding mixtures of fatty acids of preferably purely vegetable origin and/or esters thereof with lower alcohols, the natural distribution of the saturated and the mono- and polyolefinically unsaturated hydrocarbon residues being largely retained, and subsequent ethoxylation.

3. Fatty alcohol ethoxylates as claimed in claims 1 and 2, characterized in that they have been produced by reaction of the corresponding fatty alcohols at elevated temperature and in the presence of such large amounts of basic alkali metal compounds as catalysts that salt phase is precipitated as an undissolved solid phase in the neutralization step, the neutralization step having been carried out in such a way that the salt phase is precipitated in the presence of fine-particle solids dispersed in the reaction product.

4. The use of the fatty alcohol ethoxylates claimed in claim 1 as a nonionic surfactant component in liquid formulations.

## Revendications

1. Ethoxylates d'alcools gras d'origine naturelle de formule générale R¹0(CH₂CH₂O)ₓH dans laquelle x représente un nombre compris entre 2 et 10, et R¹ correspond à des mé-langes de radicaux d'hydrocarbures d'alcools gras saturés ou à insaturation oléfinique, dans la plage des C₆ à C₂₀ ou des C₁₂ à C₂₀, qui correspondent pour la plage de longueur de chaîne R¹ = C₆ à C₂₀ à la spécification I ci-après, et pour la plage de longueur de chaîne R¹ = C₁₂ à C₂₀ à la spécification II ci-après :
| De structure analogue à | | I % en poids | II % en poids |
|---|---|---|---|
| C₆ | Acide capronique | 0,1 à 0,6 | |
| C₉ | Acide caprylique | 2,5 à 10 | |
| C₁₀ | Acide caprique | 2,5 à 14 | |
| C₁₂ | Acide laurique | 37 à 52 | 39 à 69 |
| C₁₄ | Acide myristique | 10 à 20 | 10 à 27 |
| C₁₆ | Acide palmitique | 6 à 10 | 6 à 14 |
| C₁₈ | Acide stéarique | 1 à 5 | 1 à 7 |
| C_{18'} | Acide oléique | 5 à 23 | 6 à 31 |
| C_{18''} | Acide linoléique | 1 à 4 | 1 à 6 |
| C_{18'''} | Acide linolénique | 0,1 X 1 | 0,1 à 2 |
| C₂₀ | Acide arachique | 0,1 à 1 | 0,1 à 2 |

2. Ethoxylates d'alcools gras selon la revendication 1, caractérisés en ce qu'
ils sont obtenus par hydrogénation sélective de graisses et d'huiles végétales ou de mélanges correspondants d'acides gras, de préférence d'origine purement végétale et/ou de leurs esters avec des alcools gras inférieurs en conservant le plus largement possible la répartition naturelle des radicaux hydrocarbonés saturés ou comportant une mono- ou polyinsaturation oléfinique, suivie le cas échéant d'une éthoxylation, de préférence en présence de catalyseurs alcalins.

3. Ethoxylates d'alcools gras selon l'une quelconque des revendications 1 et 2,
caractérisés en ce qu'
on les a produits par mise en réaction des alcools gras correspondants à température accrue et en présence de quantités accrues de composés de métal alcalin basique comme catalyseur, de façon que, dans l'étape de neutralisation, survient une précipitation de sel en tant que phase solide non dissoute, l'étape de neutralisation étant conduite de sorte que la précipitation de la phase saline est effectuée en présence de matières salines finement divisées dispersées dans le produit réactionnel.

4. Utilisation des éthoxylates d'alcools gras selon la revendication 1,
comme composants tensioactifs non ioniques dans les formulations liquides.
